Europäisches Patentamt

European Patent Office

Office européen des brevets

(11) Publication number : **0 438 354 A1**

# EUROPEAN PATENT APPLICATION

(21) Application number : 91400097.1

(51) Int. Cl.⁵ : **C12N 5/04**

(22) Date of filing : 17.01.91

(30) Priority : 17.01.90 JP 6370/90

(43) Date of publication of application :
24.07.91 Bulletin 91/30

(84) Designated Contracting States :
AT BE CH DE DK ES FR GB GR IT LI LU NL SE

(71) Applicant : SHISEIDO COMPANY LIMITED
7-5-5 Ginza
Chuo-ku Tokyo (JP)

(72) Inventor : Gozu, Yoko, c/o Shiseido
Laboratories
1050 Nippa-cho, Kohoku-ku
Yokohama-shi, Kanagawa (JP)
Inventor : Yokoyama, Mineyuki, c/o Shiseido
Laboratories
1050 Nippa-cho, Kohoku-ku
Yokohama-shi, Kanagawa (JP)
Inventor : Satake, Motoyoshi
5-501-1004,Matsushiro
Tsukuba-shi, Ibaraki (JP)
Inventor : Shimomura, Koichiro
5-528-204,Matsushiro
Tsukuba-shi, Ibaraki (JP)

(74) Representative : Rinuy, Santarelli
14, avenue de la Grande Armée
F-75017 Paris (FR)

(54) Protoplasts of a plant of the genus Iris and methods of isolating and culturing same.

(57) Disclosed are protoplasts of a plant of the genus Iris and methods of isolating and culturing same. The method of isolating the protoplasts of a plant of the genus Iris comprises transplanting lumps of tissue of a plant of the genus Iris induced in a plant tissue-culturing solid medium into a cell-culturing liquid medium to form fine and spherical cells, and converting the thus grown fine and spherical cells to protoplasts, and the method of cultureing the protoplasts comprises culturing the protoplasts in a liquid medium containing an auxin, a carbon source, and an osmotic pressure-adjusting agent.

EP 0 438 354 A1

## PROTOPLASTS OF A PLANT OF THE GENUS IRIS AND METHODS OF ISOLATING AND CULTURING SAME

This invention relates to a protoplast culture system of a plant of the genus Iris, more specifically to protoplasts of a plant of the genus Iris, a method of isolating same and a method of culturing same.

In the breeding of a plant or the production of useful substances utilizing plant tissue culture, techniques such as cell fusion and gene introduction are widely used, and to utilize these techniques, a protoplast culture system must be established for each plant species. Regarding the culture of protoplasts, since a report was made on tobacco by Takebe et al. (Planta, 99, 12-20, 1971), the culture of protoplasts has been reported for various plant species. Recently, even successful examples of the culture of protoplasts of a rice plant, once thought to be difficult, has been accomplished (Bio. Technology, 4, 1085-1090, 1986 ; Plant Cell Reports 5, 85-88, 1986). Nevertheless, the culture methods and culture conditions are different for different plant species, and a culture method which went well for one species is not always applicable to another species.

Plants of the genus Iris are important perfume plants which are utilized not only for flower beds and cut flowers but also for accumulating orris oil, a kind of high-grade natural essential oil, at the rhizome part in some species. It is common among plants that the protoplasts thereof generally have a remarkably lower cell biological activity, and thus no successful example of the culture of protoplasts of plants of the genus Iris has been reported to date.

When breeding a plant of the genus Iris, the development of a protoplast culture system which offers a certain diversity is desired, and thus the object of this invention is to provide protoplasts of a plant of the genus Iris, a method of isolating same and a method of culturing same.

Namely, the first embodiment of the present invention relates to protoplasts of a plant of the genus Iris ; the second embodiment of the present invention relates to a method of isolating protoplasts, and comprises transplanting lumps of tissue of a plant of the genus Iris induced in a plant tissue-culturing solid medium into a cell-culturing liquid medium to form fine and spherical cells, and converting the spherical cells to protoplasts; and the third embodiment of the present invention relates to a method of culturing the protoplasts, and comprises culturing the protoplasts in a liquid medium containing an auxin, a carbon source, and an osmotic pressure adjusting agent.

## DESCRIPTION OF THE PREFERRED EMBODIMENTS

In the present invention plants of the genus Iris include all plants applicable to the invention and belonging to the genus Iris, and specific examples thereof include Iris palida, Iris florentina, Iris germanica, Iris variegata, Iris aphylla, Iris Kochii, Iris cengialti, Iris amas, Iris trojana, Iris cyperiana, Iris mesopotamica, Iris susiana, Iris Korolkowii, etc. Protoplasts of such plants of the genus Iris can be isolated by a method of isolating protoplasts, as in the second embodiment of the present invention.

Although any of the rhizome parts, flower parts and juvenile leaf parts of a plant of the genus Iris can be used in tissue culture in the second embodiment in a perfume-producing plant preferably the rhizome parts are used, particularly the base part of the leaf. Induction of lumps of tissue from pieces of such a plant can be carried out by placing these pieces, after conventional sterilization, on a plant tissue-culturing solid medium to which an auxin and, if necessary, a cytokinin have been added. Any plant tissue-culturing solid medium can be used, and examples thereof include the media of Linsmaier & Skoog (hereinafter referred to as LS), White, Ganborg, Nitsch, Heller, Schenk & Hildebrant, Nitsch & Nitsch, Kohlenbach & Schmidt, and Murashige & Skoog. Light is not required for the callus induction.

The temperature is generally from 15°C to 30°C, preferably 20°C to 30°C, more preferably 25°C to 27°C. AS the auxin, any auxins can be added, such as 2,4-dichlorophenoxyacetic acid (hereinafter abbreviated as 2,4-D), α-naphthaleneacetic acid, indole-3-acetic acid, indole-3-butyric acid (hereinafter abbreviated as IBA) and 2,4,5-trichlorophenoxyacetic acid. As the cytokinin, any cytokinins can be added, such as zeatin, 6-benzyladenine, kinetin (hereinafter abbreviated as KT), ribosylzeatin and isopentenyladenine. Preferably the auxin used is 2,4-D, and preferred as a cytokinin used is KT. The concentration of the auxin to be added is generally from 0.1 μg/ml to 10 μg/ml, preferably 0.5 μg/ml to 2 μg/ml. When the auxin concentration is below 0.1 μg/ml, the probability that callus well be induced becomes very low, and when the auxin concentration is above 10 μg/ml, the induction rate of the lumps of tissue becomes low. The addition of a cytokinin promotes the conversion to callus, and the concentration of the cytokinin is generally from 0.01 μg/ml to 1 μg/ml, preferably 0.01 μg/ml to 0.5 μg/ml. The induced callus-like tissue is then subcultured at an interval of about once a month, under the same conditions as used in induction thereof. The thus obtained lumps of tissue are large and are coagulated on the medium, and thus are unsuitable for the isolation of protoplasts. Therefore, the lumps of tissue induced and subcultured in the solid medium must be to transplanted into a liquid suspension culture cell-culturing medium to form fine and spherical lumps of cells. This is an important characteristic of the prevent invention. Preferably, the lumps of tissue are cultured, before the liquid culture, in a solid medium capable of forming relatively fine lumps of cells ; for example, the modified B 5 solid medium (the components thereof are shown in Table 1 of the later-described Example 3). The modified B 5 medium is mentioned as an example of the liquid suspension culture cell-culturing medium. By continuing the selection of such fine lumps of cells, cells can be obtained which smoothly grow as liquid culture cells. These cells are subcultured by transplanting into a fresh medium once a week, and those showing a flourishing growth can be used as a material for the isolation of protoplasts.

The convention to protoplasts can be carried out by a method known per se. For example, protoplsts of a plant of the genus Iris can be obtained by slowly shaking fine and spherical lumps of cells derived from callus, for 2 to 3 hours in an enzyme solution which contains cellulase and petctinase and in which the osmotic pressure is adjusted with mannitol.

The thus obtained protoplsts can be used, after washing with a washing liquid, for culturing according to the third embodiment of the present invention.

It is not always necessary to add a cytokinin as a plant hormone to a protoplast culture medium used in the present invention. A preferred example of such a medium contains 0.5 to 5.0 mg/l, preferably 1.0 to 2.0 mg/l of an auxin, and 1 to 10%, preferably 3 to 6% of a carbon source, and the osmotic pressure thereof has been adjusted to 0.3 to 0.7 M, preferably 0.4 to 0.5 M, with mannitol. As the auxin, there can be added 2,4-dichlorophenoxyacetic acid (hereinafter abbreviated as "2,4-D"), α-naphthaleneacetic acid (hereinafter abbreviated as "NAA"), indole-3-acetic acid, indole-3-butyric acid, 2,4,5-trichlorophenoxyacetic acid or the like. Glucose or sucrose can be used as a carbon source. Preferable if the osmotic pressure is too low, due to the use of glucose or sucrose, it is adjusted by an osmotic pressure adjusting agent, for example, a metabolically inactive saccharide such as mannitol or sorbitol. Any medium, other than a liquid suspension culture cell-culturing medium, i.e. the modified B 5 medium, can be used as a basal medium in the present invention, and examples thereof include MS, LS, V-47, VKM, B 5, KM8P, and WB media. In the case of protoplasts of a plant of the genus Iris, embedding same in the medium inhibits the growth of the protoplasts, and thus they are preferably cultured in a liquid medium or the embedded cells are floated in a liquid medium. When the protoplasts are cultured, for example, at a temperature of about 20 to 30°C under a dark condition or under illumination and under a stationary condition or with slow shaking at a revolution speed of 40 to 50 rpm, many colonies are formed after about 40 to 50 days. The thus obtained colonies are transplanted to an appropriate medium and then subjected to callus formation according to a conventional method, respectively.

[Examples]

The present invention is now specifically described below by examples, but the scope of the invention is not limited thereby.

Example 1 Preparation of lumps of tissue

The rhizome of Iris pallida was cut off at the base of a leaf, and after water washing, cut up to form 1 cm³ pieces and immersed in 70% ethanol for several minutes. After again water washing, the pieces were immersed in a 5% sodium hypochlorite solution for 20 minutes, and, after a further water washing, cut again to form pieces having a thickness of 1 to 2 mm. After another immersion in a 1% sodium hypochlorite solution for 10 minutes, the pieces were further cut to form 2 mm square pieces, and placed on the LS solid medium containing 1.0 μg/ml of 2,4-D and 0.1 μg/ml of KT. The pieces were then cultured at 26°C in a dark place, and lumps of tissue appeared after about one month. The obtained culture was subcultured by transplanting at an interval of about once a month under the same conditions, to obtain large lumps of tissue which are coagulated.

Example 2 Isolation of protoplasts

When the lumps of tissue obtained as in Example 1 were transplanted to the modified B 5 solid medium, which was completely different from the LS medium in composition, the appearance of fine and spherical lumps of cells was observed. The fine and spherical lumps of cells were then cultured for about 4 months in a fresh modified B 5 liquid medium, and fine and spherical lumps of cells suitable of liquid culture were selected, to thereby establish a liquid culture system. The liquid culture cells were subcultured once a week, and the liquid suspension culture cells showing a flourishing growth were used as a material for the isolation of protoplasts. To the thus obtained liquid suspension culture cells was added an enzyme solution which contained cellulase and pectinase, and in which the osmotic pressure was adjusted to 0.5 M with mannitol, and the mixture was slowly shaken at 27°C in a dark place. After an enzyme treatment for 2 to 3 hours, the mixture was filtered with a 40 μm nylon mesh to remove untreated lumps of cells and cell debris, and the filtrate was centrifuged to separate the enzyme solution and the protoplasts. The obtained protoplasts were washed three times with a washing solution having the following composition, and then used for the next culture.

| | |
|---|---|
| $KH_2PO_4$ | 27.2 mg/$\ell$ |
| $KNO_3$ | 101.0 mg/$\ell$ |
| $CaC\ell_2 \cdot 2H_2O$ | 1480 mg/$\ell$ |
| $MgSO_4$ | 240.0 mg/$\ell$ |
| KI | 0.16 mg/$\ell$ |
| $CuSO_4 \cdot 5H_2O$ | 0.025 mg/$\ell$ |
| Mannitol | 90 g/$\ell$ |

Example 3 Culture of protoplasts

Basal media having the respective compositions shown in the following Table 1 were prepared, a plant hormone and a carbon source were added to each basal medium as shown in Table 2, and then the osmotic pressure of the resulting solutions then adjusted with mannitol to prepare plant-culturing media. The protoplasts obtained in Example 2 were suspended in each of the thus prepared media to a cell concentration of $10^4$ cells/ml to $10^6$ cells/ml, and the mixtures were cultured at 27°C in a dark place, respectively.

As the culture methods, a liquid culture method, a solid culture method and a liquid-solid culture method were employed. Although the growth of protoplasts was inhibited in the solid culture method, and the appearance of colonies was not observed, in the liquid culture method and the liquid-solid culture method, the protoplasts started the first division 2 to 3 days after the start of the culture and grew into colonies on the order of 0.2 to 0.5 mm, 1 to 2 months thereafter. The frequency of appearance of colonies at that time was as shown in Table 3. When the thus appeared colonies were transplanted into a conventional callus-subcolturing medium (a Linsmaier & Skoog agar medium to which 1 ppm of 2,4-D and 0.1 ppm of kinetin were added), they smoothly grew to form callus.

## Table 1

| Components of the basal medium | Basal medium 1 (Modified B5 medium) | Basal medium 2 (LS medium) | Basal medium 3 (KM8P medium) |
|---|---|---|---|
| $NH_4NO_3$ | – | 1650 | 600 |
| $KNO_3$ | – | 1900 | 1900 |
| $CaCl_2 \cdot 2H_2O$ | 150 | 440 | 600 |
| $MgSO_4 \cdot 7H_2O$ | 250 | 370 | 300 |
| $KH_2PO_4$ | – | 170 | 170 |
| $KCl$ | 2950 | – | 300 |
| $FeSO_4 \cdot 7H_2O$ | 28 | 28 | 28 |
| $Na_2EDTA$ | 37 | 37 | 37 |
| $H_3BO_3$ | 3 | 6.2 | 3 |
| $MnSO_4 \cdot H_2O$ | 10 | 22.3 | 10 |
| $ZnSO_4 \cdot 7H_2O$ | 2 | 8.6 | 2 |
| $NaMoO_4 \cdot 2H_2O$ | 0.25 | 0.25 | 0.25 |
| $CuSO_4 \cdot 5H_2O$ | 0.025 | 0.025 | 0.025 |
| $CoCl \cdot 6H_2O$ | 0.025 | 0.025 | 0.025 |
| KI | 0.75 | 0.83 | 0.75 |
| Inositol | 100 | 100 | 100 |
| Nicotinic acid | 1 | 0.5 | 1 |
| Pyridoxine-HCl | 1 | 0.5 | 1 |
| Thiamine·HCl | 10 | 0.4 | 10 |
| Calcium pantothenate | – | – | 1 |

Table 1 (continued)

| Components of the basal medium | Basal medium 1 (Modified B5 medium) | Basal medium 2 (LS medium) | Basal medium 3 (KM8P medium) |
|---|---|---|---|
| Folic acid | – | – | 0.4 |
| Aminobenzoic acid | – | – | 0.02 |
| Biotin | – | – | 0.01 |
| Choline chloride | – | – | 1 |
| Accorbic acid | – | – | 2 |
| Vitamin A | – | – | 0.01 |
| Vitamin $D_3$ | – | – | 0.01 |
| Vitamin $B_{12}$ | – | – | 0.02 |
| Glycine | 7.5 | – | 2 |
| Sodium pyruvate | – | – | 20 |
| L-Glutamine | 876 | – | – |
| L-Aspartic acid | 266 | – | – |
| L-Arginine | 174 | – | – |
| Citric acid | – | – | 40 |
| Sodium malate | – | – | 40 |
| Fumaric acid | – | – | 40 |
| Casein hydrolyzate | – | – | 250 |
| $(NH_4)_2SO_4$ | 134 | – | – |
| $NaH_2PO_4 \cdot H_2O$ | 150 | – | – |

(mg/ℓ)

EP 0 438 354 A1

## Table 2

| Basal medium | Plant hormone | Glucose concentration | No. |
|---|---|---|---|
| 1 | NAA1.5 (ppm) | 3% | 1 |
| 2 | NAA1.5 | 3% | 2 |
| 3 | NAA0.5 | 3% | 3 |
| 3 | NAA1.5 | 3% | 4 |
| 3 | NAA1.5 | 9% | 5 |
| 3 | NAA3.0 | 3% | 6 |

## Table 3

| Medium | Colony appearance frequency |
|---|---|
| 1 | 0 |
| 2 | 0.08 |
| 3 | <0.01 |
| 4 | 0.16 |
| 5 | <0.01 |
| 6 | <0.01 |

## Claims

1. Protoplasts from a plant of the genus Iris.

2. A method of isolating protoplasts which comprises transplanting lumps of tissue of a plant of the genus Iris induced in a plant tissue-culturing solid medium into a cell-culturing liquid medium, and converting the spherical cells to protoplasts.

3. A method of culturing the protoplasts of claim 1, which comprises culturing the protoplasts in a liquid medium containing an auxin, a carbon source and an osmotic pressure adjusting agent.

7

))) European Patent Office

# EUROPEAN SEARCH REPORT

Application Number

| | DOCUMENTS CONSIDERED TO BE RELEVANT | | EP 91400097.1 |
|---|---|---|---|

| Category | Citation of document with indication, where appropriate, of relevant passages | Relevant to claim | CLASSIFICATION OF THE APPLICATION (Int. Cl.5) |
|---|---|---|---|
| D,A | PLANTA, vol. 99, 1971, Berlin<br>T. NAGATA et al. "Plating of Isolated Tobacco Mesophyll Protoplasts on Agar Medium"<br>pages 12-20<br> * Totality *<br>-- | 1,2 | C 12 N 5/04 |
| A | DD - A1 - 263 077<br>(PÄDAGOGISCHE HOCHSCHULE "KARL LIEBKNECHT" POTSDAM)<br> * Abstract *<br>-- | 1,2 | |
| A | EP - A2 - 0 202 667<br>(MITSUI TOATSU CHEMICALS INC.)<br> * Abstract *<br>-- | 1,2 | |
| P,A | PATENT ABSTRACTS OF JAPAN,<br>unexamined applications,<br>C field, vol. 14, no. 423,<br>September 12, 1990<br>THE PATENT OFFICE JAPANESE GOVERNMENT<br>page 117 C 757<br> * Kokai-No. 2-163 017<br>(SHISEIDO CO LTD) *<br>---- | | TECHNICAL FIELDS SEARCHED (Int. Cl.5)<br><br>C 12 N |

The present search report has been drawn up for all claims

| Place of search | Date of completion of the search | Examiner |
|---|---|---|
| VIENNA | 14-03-1991 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document